(19) Europäisches Patentamt
European Patent Office
Office européen des brevets

(11) Publication number: 0 302 442
A2

# (12) EUROPEAN PATENT APPLICATION

(21) Application number: **88112510.8**

(22) Date of filing: **02.08.88**

(51) Int. Cl.4: **C12P 21/00 , C12N 1/20 , //C07K5/00**

(30) Priority: **07.08.87 US 83741**

(43) Date of publication of application:
**08.02.89 Bulletin 89/06**

(84) Designated Contracting States:
**AT BE CH DE ES FR GB GR IT LI LU NL SE**

(71) Applicant: **W.R. Grace & Co.-Conn. (a Connecticut corp.)**
**Grace Plaza 1114 Avenue of the Americas**
**New York New York 10036(US)**

(72) Inventor: **Lovinger, Gerald George**
**18606 Walkers Choice Road**
**Gaithersburg Maryland 20879(US)**
Inventor: **Gross, Akiva Tuvia**
**2441 McCormick Road**
**Rockville Maryland 20850(US)**
Inventor: **Fortney, Donald Zane**
**1555 Clairidge Road**
**Baltimore Maryland 21207(US)**

(74) Representative: **UEXKÜLL & STOLBERG**
**Patentanwälte**
**Beselerstrasse 4**
**D-2000 Hamburg 52(DE)**

## (54) Vibriolysin coupling process.

(57) Disclosed herein is an enzymatic process to bind two amino acids in the presence of a water-miscible solvent. Preferably the amino acids are precursors of aspartame and a preferred solvent is acetonitrile. The enzyme is vibriolysin.

EP 0 302 442 A2

# VIBRIOLYSIN COUPLING PROCESS

## Background of the Invention:

Enzyme mediated synthesis of dipeptides is well known. Thus U.S. Patents 4,165,311; 4,436,925 and 4,256,836 describe synthesis in aqueous medium to form insoluble addition compounds, e.g., the addition compound of one mol of phenylalanine methyl ester with one mol of N-protected aspartyl-phenylalanine methyl ester. U.S. Patent 4,284,721 teaches that N-protected aspartic acid and phenylalanine lower alkyl esters can be enzymatically coupled in the presence of a water-immiscible solvent which can contain water-miscible co-solvents, but the amount of the water-miscible solvent must be limited to avoid inactivating or inhibiting the enzyme. U.S. Patents 4,116,768 and 4,119,493 contain similar teachings with regard to the use of water-miscible solvents as co-solvents in aqueous medium. Similarly Angew. Chem. Int. Ed. Engl. 24 (1985) Number 2, page 87, indicates that water-miscible solvents can be used as co-solvents in admixture with water but that the catalytic activity of protease enzymes decreases as the concentration of the co-solvent increases and that at 50% and above no synthesis occurs where chymotrypsin is used as the enzyme. As a possible exception, the use of a polyol (e.g., 1,4-butanediol) may in some cases stabilize the enzyme. The use of aqueous or aqueous-organic media for enzymatic coupling to form N-formyl dipeptides (e.g., N-formyl aspartame) and polypeptides is also described in WO 8604924 and European Patent Publication 0149594.

A number of articles in various scientific journals also discuss the use of enzymes with a combination of water and water-miscible organic solvents and obtain yields which appear to vary depending upon the choice of solvent, amount of water, enzyme and substrate. Also, whether the enzyme is immobilized appears to be a factor. The use of a 50/50 acetonitrile/water solvent system is described by Nilsson and Mosbach in Biotech Bioeng. 26, 1146 (1984). This reference also describes the use of butanediol/water (90/10). The use of acetonitrile as a solvent is also discussed by J.B. Jones, and J.F. Beck, in Applications of Biochemical Systems in Organic Chemistry, Part 1 (J.B. Jones, C.J. Sih. and D. Perlman, eds.) p. 107 ff. New York; J. Wiley, 1976; and J.B. Jones and M.M. Mehes., Can. J. Chem. 57, 2245 (1979). The coupling of L-phenylalanine methyl ester (i.e., L-pheOMe) and N-protected N-carbobenzyloxy-aspartic acid (i.e., Z-asp) using a mixture of water-immiscible/water miscible solvents is described in Biotech. Lett. 7, 789 (1985). Konnecke et al. in Monatshrifts fur Chemie, 112, 469-481 (1981) at page 475 refer to the use cf acetonitrile as a solvent. Other articles of interest are J. Biochem., 89, 385 (1981); J. Org. Chem., 51, 2728(1986); Coll Czechos. Chem. Comm., 49, 231 (1984); and Proc. Natl. Acad. Sci., 80, 3241 (1983).

While the literature indicates that enzymes in general and proteases in particular have been employed in both water-miscible and water-immiscible organic solvents, there appears to be a general conception that the water-miscible solvents are somewhat inferior. Thus it has been said that "most enzymes are inactive in hydrophilic, water-miscible organic solvents, which is easy to understand in terms of partitioning of the essential water from the enzyme into them." (A.M. Klibanov, Chemtech, page 354, June 1986).

This invention is based on the use of proteases in water-miscible organic solvents

## Description of the Invention:

The invention is a process wherein a metalloendoprotease enzyme is used to catalyze peptide bond formation between two substrates selected from the group consisting of N-substituted aspartic acid and a phenylalanine lower alkyl ester. The benzylic carbon atom of the ester can be substituted with one or more labile groups readily replaceable with hydrogen. Preparation of the enzyme at elevated pH levels improves the activity of the protease and significantly diminishes competing esterase activity. The process of the invention encompasses carrying out the above process in the presence of a water-miscible organic solvent.

The use of water-miscible solvents offers many advantages. Contrary to expectations the solvent can be employed without depleting the enzyme of essential water. For example in conducting a continuous process the amount of water essential for enzyme activity can be provided by maintaining from 2-10% by weight of the solvent system of water with the balance being the water-miscible solvent or a mixture thereof with other solvents. In a closed system (e.g., essentially a batch reaction as opposed to a continuous reaction) the enzyme and its support will lose enough water to provide the 2-10% by weight referred to above. It should be understood however that if the enzyme is contacted with a sufficiently large amount of essentially anhydrous water-miscible solvent, enough water will be extracted so that the water level in the solvent falls

below about 2% and the enzyme is denatured. Amounts of water in excess of 10%, e.g., as high as 50%, can be employed but in doing so the advantages of using the water-miscible solvent may be reduced.

In many reactions the use of a water-miscible solvent as the sole solvent or as a co-solvent can provide a single liquid phase, thereby avoiding phase transfer limitations which arise where the solvent is immiscible with water. For example, use of a water-miscible solvent will frequently increase the reaction rate. Also the dielectric constant of most useful water-miscible organic solvents is from 5 to 60 (preferably 30 to 60) which contributes to formation of a single liquid phase because most amino acid derivatives are relatively polar and are soluble in such solvents. For example the methyl ester of phenylalanine is much more soluble in acetonitrile than in hexane or ethyl acetate.

The use of a water-miscible solvent can also shift the reaction equilibrium. For example, in ethyl acetate the reaction of N-formyl aspartic acid with phenylalanine methyl ester gives a yield of about 10%, but the yield is about 80% in acetonitrile.

The enzyme, whether immobilized or in "free" form, may be much more stable in water-miscible solvent as opposed to a water-immiscible solvent. By "stability" it is meant that the enzyme resists denaturation whether by extraction of water or other means. Also the term "solvent system" is used to designate the solvent portion of the liquid phase, and includes the water-miscible solvent and any co-solvents utilized therewith, e.g., water or a water-miscible solvent.

By the term "water-miscible organic solvent" is meant those organic liquids which are miscible with water in any proportion to form a single phase system. Examples of suitable organic solvents include alcohols (e.g., ethanol, 1-propanol, and 2-propanol); polyols (e.g., 1,4-butanediol, and diethylene glycol); nitriles (e.g., acetonitrile); and ethers (e.g., dioxane and tetrahydrofuran); as well as other solvents such as dimethylformamide, dimethyl sulfoxide, and acetone.

Acetonitrile is a preferred water-miscible solvent. A further embodiment of the invention is that acetonitrile can be used with a wide variety of amino acids and enzymes to form dipeptides and polypeptides by an enzyme mediated coupling reaction. Examples of suitable amino acids for use in this reaction include aliphatic amino acids such as monoamino monocarboxylic acids, e.g., glycine (Gly), alanine (Ala), valine (Val), norvaline (nor-Val), leucine (Leu), isoleucine (iso-Leu), norleucine (nor-Leu); oxyamino acids, e.g., serine (Ser), threonine (Thr), homo-serine (homo-Ser); sulfur-containing amino acids, e.g., methionine (Met) or cystine (CysS) and cysteine (CysH); monoamino dicarboxylic acids, e.g., aspartic acid (Asp) and glutamic acid (Glu); diamino monocarboxylic acids, e.g., ornithine (Orn), lysine (Lys), arginine (Arg); aromatic amino acids, e.g., phenylalanine (Phe), tyrosine (Tyr) and heterocyclic amino acids, e.g., histidine (His), tryptophan (Trp). (The amino acids are designated by symbols which are commonly used in the field.)

Care should be used in solvent selection. For example, if the enzyme contains a metal, the solvent should not complex the metal. DMF and DMSO appear to complex the metal component in metalloproteinases and should preferably be restricted to 50% or less (on a molar basis) of the solvent system, e.g., the balance can be water or another solvent. The solvent should also be inert in the sense that it does not chemically react with the enzyme or the substrates. For example, if acetone is the solvent, it should be used under conditions which minimize reaction with the amine groups of a substrate or an enzyme.

The amino acids functioning as the acyl donor generally have a protective group at the N position Examples of suitable N-protective groups are those normally used in peptide synthesis such as tertiary alkoxycarbonyl groups, e.g., t-butyloxylcarbonyl (BOC-), t-amyloxycarbonyl (t-Aoc); benzyloxycarbonyl groups which can be substituted with an inert substituent, such as benzyloxycarbonyl (Z-), p-methoxybenzyloxycarbonyl (PMZ-), 3,5-dimethoxybenzyloxycarbonyl ($Z(OMe)_2$-), 2,4,6-trimethylbenzyloxycarbonyl (TMZ-), p-phenylazobenzyloxycarbonyl (PZ-), p-toluenesulfonyl (tosyl-); o-nitrophenyl sulfenyl (Nps-), and the like. Also formyl can be employed.

Examples of suitable amino acids for donating the amino moiety in formation of a dipeptide or polypeptide include any of those set forth above. Phenylalanine is preferred, and particularly derivatives substituted at the benzylic carbon can be employed, e.g., derivatives wherein the benzylic carbon is substituted with at least one group easily replaceable by methods such as catalytic hydrogenolysis or electrochemical reductive cleavage. Examples of suitable substituted phenylalanine derivatives include those corresponding to the formula:

$$
\begin{array}{c}
Ph \\
| \\
H-C-X \\
| \\
H-C-NH_2 \\
| \\
CO_2R
\end{array}
$$

wherein Ph is phenyl (substituted or unsubstituted), while X is -OH, -SH, -Cl, -Br, -I, $-OCOCH_3$, $-OCOOCH_3$, $-NH_2$ or $-SCH_3$ and R is a lower alkyl group having from 1 to 4 carbon atoms.

The amino-donating amino acids are protected by suitable C-terminal protective groups. The protective groups for the carboxyl group (C-terminal protective groups) of the amine component include alkoxy groups such as methoxy (-OMe), ethoxy (-OEt); tertiary alkoxy groups such as t-butoxy (-O-t-Bu); and benzyloxy groups which can be substituted such as benzyloxy (-OBzl), p-nitrobenzyloxy (-OBzl(p-$NO_2$)), benzhydryloxy (-OBzh), benzylamino (-NHBzl), 2,4-dimethoxybenzylamino (-NHDMB), benzhydrylamino (-NHBzh) or unsubstituted amino ($-NH_2$), etc. Also the amide and hydrazide groups can be employed as C-terminal protective groups.

It should be understood that the water-miscible organic solvents can be utilized either in essentially anhydrous "neat" form or in conjunction with water and/or other organic solvents (both water-immiscible and water-miscible solvents). Where water is employed, the amount should generally be less than 50% by weight based on the total solvent system (e.g., water plus water-miscible solvent). However, certain solvents appear to complex metal ions and thereby inactivate various metalloproteinase enzymes, and the amount of water used with such solvents should equal or exceed 50% by weight of the solvent system. Examples of complexing solvents include DMF and DMSO. If the solvent is in dry or neat form, it will contain some water (e.g., up to about 10% by weight of the solvent) which has been given up by the support used to immobilize the enzyme. For the preferred acetonitrile solvent, generally the amount of water is kept at a minimum and "neat" acetonitrile has been found to be a good solvent, in which case the only water provided will be that coming from the support. However when run on a continuous basis the water level in the acetonitrile solvent should be maintained at a level of at least 10 weight % and generally will fall within the range of from 5 to 50 weight %. This amount of water is advisable to avoid dehydrating the enzyme and can be of assistance in dissolving substrates. If desired, the water can be added via the substrate stream if the process is practiced on a continuous basis. Specifically in coupling N-protectedaspartic acid with phenylalanine lower alkyl esters and benzyl substituted derivatives thereof (whether via a batch or continuous process), the acetonitrile can also be used with varying amounts of water, but it is preferred that the amount of water be less than 50 weight percent, i.e., by weight the $CH_3CN/H_2O$ ratio should exceed 1, and preferably should exceed about 2.5.

Using procedures known to those skilled in the art, the organic solvent selected for each coupling reaction can be optimized in view of a number of factors such as the solubility of the substrates and dipeptide or polypeptide product in the solvent, the amount of water or other co-solvent present, the effect of the co-solvent upon the enzyme and other factors.

Metalloproteases are known to mediate peptide bond formation. In this invention, the metalloendoprotease from Vibrio proteolyticus ATCC 53559 is used with the water-miscible organic solvent system described above. This protease has been designated "vibriolysin" and will be referred to herein by that name. Other strains of V. proteolyticus may be available from ATCC or from private sources, but may not produce the desired enzyme. The V. proteolyticus ATCC 53559 strain has been deposited with the American Type Culture Collection, 12301 Parklawn Drive, Rockville, Maryland 20852. The culture was deposited with no restrictions as to availability and W. R. Grace & Co., the assignee in this case, assures permanent availability of the culture to the public through ATCC upon granting of the patent now applied for. The enzyme need not be used in purified form but may be a more or less crude preparation (e.g., a concentrated partially purified fermentation broth) containing the vibriolysin enzyme and which may also contain one or more other enzymes.

As is well known many protease enzymes also exhibit esterase activity. This activity can be decreased on occasion by appropriate selection of the organic water-miscible solvent. For example acetonitrile has exhibited some effect in reducing esterase activity. Also it is possible to use an inhibitor to further decrease esterase activity if the esterase activity is contributed by a separate protein or if the site for esterase activity differs from the protease site, assuming both sites are on the same molecule. Suitable inhibitors can be extracted from oats, fava, kidney beans and potato. The method of extraction is disclosed in Nippon Nogei

Kogaku Kaishi, 31, page 38(1957). The inhibitor need not be a pure material, but can be a crude extract.

Alternatively, the esterase activity present in vibriolysin preparations can be diminished by production of the enzyme under conditions of elevated pH. By culturing Vibrio proteolyticus ATCC 53559 at a pH of about 8.0 to about 8.6, preferably about 8.4 to about 8.6, it is possible to harvest the vibriolysin enzyme with little or no contaminating esterase. Preparation at the elevated pH taught here results in both a decrease in production of the undesirable esterase activity and an increase in production of the desired protease (vibriolysin) activity. With the esterase activity thus diminished, the vibriolysin can be utilized in either crude or purified form as described herein.

The protease enzyme can be used in this invention in either bound or free form. By the term "bound" it is meant that the enzyme is immobilized on a suitable insoluble carrier to form a complex which can be recovered and reused. Suitable methods of immobilization include physical adsorption, ionic bonding, covalent bonding, crosslinking following adsorption or other methods of inclusion of the enzyme in or on a supporting material essentially insoluble in the reaction medium. Suitable substrates include siliceous materials (e.g., porous silica), non-silicious ceramics (e.g., alumina), or natural or synthetic organic polymeric materials (e.g., resins such as Amberlite XAD-7, polyacrylamide copolymers, agarose, and alginates).

By contrast a "free" enzyme is unbound and can be either dissolved or suspended in the solvent system. The enzyme can be used as a suspended solid precipitate in acetonitrile, without first having to be bound or immobilized on a substrate as described above. The solid enzyme is prepared by precipitating in the presence of acetonitrile, as described in Example III.

It is preferable to have a high concentration of the substrate amino acids to enable the process to be carried out at a reasonable reaction rate. Each substrate for the coupling reaction is used in a concentration within the solubility thereof in the solvent. However, since the materials are consumed as the reaction proceeds, it is possible to have a portion of any substrate in a suspended state. In solution the substrate materials should each be present in a concentration ranging from about 0.001M and to about 2M, and preferably between about 0.1M and about 1M.

With regard to the N-substituted aspartic acid/phenylalanine lower alkyl ester coupling reaction, the acid/ester mole ratio can be 1:1 in mole ratio when both substrates are in L configuration. Practically, they may be used in a ratio ranging between 10:1 and 1:10 and preferably between 3:1 and 1:5. In cases where the materials are in a DL configuration, they may be used in quantities which result in a ratio of the L-isomers as described above.

The invention can be carried out, for example, by allowing a water-containing immobilized enzyme to be suspended in the organic solvent miscible with water which also contains both of the starting materials and then by allowing the reaction to proceed with stirring. Upon completion of the reaction, the immobilized enzyme and a suspension or solution containing a reaction product can be separated from each other by subjecting the product-containing medium to filtration or other separation processes.

The invention can also be carried out in a column filled with the water-containing immobilized enzyme, by allowing the water-miscible organic solvent which contains the two starting materials to flow through the column. This process permits the reaction to be continuously carried out and is advantageous for an industrial application of the invention.

The reaction temperature is usually in the range between about 10 and about 35° C. and preferably between about 20 and about 25° C.

The reaction time depends on the concentrations of the two substrates, the quantity of the immobilized enzyme, a predetermined conversion rate, etc. However, usually the reaction time of about 0.5 to about 200 hours and preferably about 2 to about 24 hours suffices.

Where the desired product is the dipeptide known as aspartame, the reaction product, i.e., N-substituted-L- aspartyl-L-phenylalanine methyl ester can be isolated by conventional means such as concentrating the reaction mixture followed by crystallization, extraction or the like. The reaction mixture can also be separated from the immobilized enzyme by suitable processes well known in the art. Following separation the immobilized enzyme can be reused.

In carrying out the invention, the amino acid substrates may be in the DL or the L configuration. Where the enzyme is specific for L isomers, if DL isomers are employed, only the L isomer participates in the reaction, while the D isomer remains unreacted in the reaction medium. If the enzyme has no stereospecific preference, the D isomer can be used, e.g., with enzymes such as serine proteases which have no D,L specificity, the amino donor (e.g., alanine or phenylalanine) can be D.

Example I. Preparation of Vibriolysin by Fermentation of Vibrio proteolyticus ATCC 53559

1. Preparation of Seed Culture

A. Preparation - 100 ml seed medium is contained in a 500 ml. indented Erlenmeyer flask and autoclaved 20 minutes at 121 °C.

B. Inoculation - A single -70 °C ampoule of organism is thawed under tap water, then aseptically transferred to the seed flask.

C. Incubation - The inoculated flask is incubated 18 hours at 250 rpm/27 °C.

D. Growth measured at 640 nm. is between an optical density of 4.0 to 6.0; broth pH is approximately 8.0.

2. Enlarged Fermentation - 1.0-liter volume in a 1.5-liter fermenter.

A. Preparation - All medium ingredients are added to the vessel (polypeptone - 40 gm., sea salts - 20 gm., $MgSO_4 \cdot 7\ H_2O$ - 0.4 gm., P-2000 - .2 ml.) and pH is unadjusted prior to sterilization; it should be nearly pH 7.0. A 1.0-liter vessel, if sterilized in an autoclave, should be sterilized 45 min. at a temperature of 121 °C.

B. Inoculation

(1) First set and double check operating parameters:

a. pH to 8.6 with 6N NaOH
b. temperature = 27 °C
c. RPM = 1000
d. dissolved oxygen readout to 100% at 1.0-LPM air.

(2) Inoculate with 10 ml. seed broth.

C. Operation

(1) Maintain aforementioned parameters.
(2) Dissolved oxygen will drop to about 75-80% at peak demand.
(3) Monitor the following:

a. Optical Density - read at 640nm Absorbance. Peaks at about 10-12 O.D. in about 12-14 hours.
b. Production of metalloendopeptidase - to about 0.1 units/sec.

3. Harvest & Concentration of Vibriolysin

At about 10-14 hours into the fermentation the product enzyme reaches titers of approximately 0.10 to 0.20 gm/liter as measured by the FAGLA assay. The broth is harvested before the cells lyse to an advanced stage (about 10-25%.

First, the whole broth is centrifuged to separate the cell portion. Then the supernatant is concentrated 70-100 fold, utilizing an Amicon SIOYIO or SIYIO ultrafilter cartridge.

The vibriolysin enzyme frequently is contaminated with one or more aminopeptidases. The undesirable esterase activity of these enzymes can be reduced by maintaining the vibriolysin concentrate at ambient temperature (e.g., 25 °C) at a pH of about 11.5 for approximately six hours, until the aminopeptidase activity fell below about 0.7 units/second (as defined below). Finally, the treated, esterase-free vibriolysin concentrate was washed with deionized water until its conductivity reading was below 1.0 mS. The aminopeptidase activity can be assayed by the following procedure:

Aminopeptidase

Aminopeptidase (esterase) activity can be determined by monitoring the increase in absorbance at 05 nm due to the release of p-nitroaniline from L-leucine-p-nitroanilide (Sigma Chemical Co., St. Louis, MO) [Prescott, J.M. et al, Biochemistry, 24:5350-5356 (1985)]. The assay is performed using 0.7 ml, 0.001 M leucine p-nitroanilide, 50 mM tris-HCl (pH 7.04) and a suitable amount of enzyme. The activity units are defined as:

Aminopeptidase Units/ml.

$$= \frac{\Delta \text{ Abs./min x (Cuvette vol. + Sample vol.) x 10}}{\text{9.66 x (Sample vol.)}}$$

where the volumes are measured in microliters and 9.66 is the micromolar extinction coefficient for L-leucine-p-nitroanalide.

Vibriolysin Activity

For determination of vibriolysin activity, azocasein (sulfanilamideazocasein, Sigma Corp., St. Louis, MO) can be used as a substrate. Vibriolysin is incubated with 50 mM tris-HCL buffer (pH 7.4) containing 1.0 mg/ml azocasein in a final volume of 0.5 milliliters. After 10 minutes incubation at 37° C, 0.5 milliliters 10% w/v trichloroacetic acid are added and immediately mixed, and the mixtures are stored on ice for 10 minutes. The mixtures are then centrifuged and the optical densities of the resulting supernatants are determined at 420 nm against a blank that contains either no enzyme or inactivated enzyme in the buffered azocasein solution.

Example II. Coupling of N-Formyl-Aspartic Acid

Fermentation broth from Vibrio proteolyticus, produced as in Example I, was concentrated and washed. The neutral protease in the fermentation broth was immobilized on Amberlite XAD-7 by the following procedure: Amberlite XAD-7 resin beads were washed with ethanol and then with water to remove fines. The washed beads were re-suspended in 0.05M MES-0.02M $CaCl_2$ solution (MES is 2[N-morpholino]-ethanesulfonic acid). After removing excess water by filtering Amberlite XAD-7 through vacuum, beads (100 grams) were suspended in 100 ml of 0.05M MES-0.02M $CaCl_2$ buffer containing 9.0 grams vibriolysin at 4° C. After shaking overnight, the immobilized vibriolysin was thoroughly washed with the MES-$CaCl_2$ buffer and then was vacuum filtered.

N-formyl-aspartic acid (1.93 g) and L-phenylalanine methyl ester (6.2 g) were dissolved in acetonitrile to give a final volume of 150 ml. After the addition of 14.5 g wet immobilized neutral protease, the reaction was run for 24 hours at room temperature. The final product concentration (N-formyl-aspartyl- L-phenylalanine methyl ester) was 11.7 g/l determined by HPLC. The solvent was evaporated in vacuum and the residue dissolved in ethylacetate and washed twice with 1N HCl. The aqueous phase was treated with acetate. The combined organic phase was washed with brine and dried over anhydrous $MgSO_4$. Evaporation of the solvent afforded a colorless solid that was crystallized from dichloroethane and identified as N-formyl-aspartame.

Example III - Use of Suspended Solid Enzyme

To a gently stirred solution of vibriolysin prepared in Example I (5.0 ml, 10-11 mg protein/ml), acetonitrile (45 ml) was added dropwise at 0° C. The resulting precipitate was collected by centrifugation. This crude enzyme preparation was added to a 95% acetonitrile solution of 80 mM formylaspartic acid and 240 mM L-phenylalanine methyl ester. The reaction mixture was stirred for 5 hours. HPLC analysis determined that the mixture contained 65 mM formylaspartame.

## Claims

1. A process for manufacturing a dipeptide or polypeptide by reacting an acid component of an amino acid or peptide having an N-terminal protective group or a salt thereof with an amine component of an amino acid or peptide having a C-terminal protective group or a salt thereof in the presence of vibriolysin to mediate peptide bond formation.

2. A process as in Claim 1 wherein the reaction is carried out in the presence of a water-miscible solvent.

3. A process as in Claims 1 or 2 wherein the solvent is acetonitrile.

4. A process as in Claims 1 to 3 wherein the solvent is at least 50 weight % acetonitrile and the balance is water, one or more water-miscible organic solvents or mixtures thereof.

5. A process as in Claim 2 wherein the water-miscible solvent is a monohydric or polyhydric alcohol, a nitrile, an ether or a mixture thereof.

6. A process as in Claims 1 to 5 wherein the amino acid components are N-substituted aspartic acid or a salt thereof and a phenylalanine lower alkyl ester wherein the benzylic carbon atom is substituted with hydrogen or one or more labile groups readily replaceable with hydrogen

7. A process as in Claim 6 wherein the N-protective group is tertiary alkoxycarbonyl, benzyloxycarbonyl, p-toluenesulfonyl, o-nitrophenylsulfonyl or formyl.

8. A process as in Claims 1 to 7 in which said vibriolysin is prepared from cultures of <u>Vibrio proteolyticus</u> ATCC 53559.

9. A process as in Claim 8 in which said vibriolysin is prepared under conditions in which the pH is between about 8.0 and about 8.6.

10. The process of Claims 1 to 9 in which said reaction temperature is between about 10 and about 35 °C.